# EUROPEAN PATENT APPLICATION

(11) **EP 2 708 555 A2**
(43) Date of publication of application: **19.03.2014**
(21) Application number: 13184790.7
(22) Date of filing: 17.09.2013
(51) Int. Cl.: C07K 16/14, G01N 33/569

(54) **Polyclonal antibodies to cloned fungal polypeptide targets**

(30) Priority: 17.09.2012 US 201261701895 P
(71) Applicant: VLN Biotech Inc., Ottawa, Ontario K1J 6X7 (CA)
(72) Inventor: Vijay, Hari M., Ottawa, Ontario K1J 6H9 (CA)
(74) Representative: Tischner, Oliver

(57) **Abstract**

Described herein are processes for preparing a polyclonal antibody which specifically binds to a cDNA derived fungal allergen. Such antibodies, for example, polyclonal antibodies specific for cloned Alt al protein from *A. alternata* or the cloned allergen Ch 2.1 of *C herbarum,* can be used in assays and kits for detecting allergens in house-hold dust.

## Description

### FIELD OF INVENTION

The present invention relates to processes for preparing a polyclonal antibody which specifically binds to a cDNA derived fungal allergen, for example, the cloned Alt a1 protein from *A. alternata* or the cloned allergen Ch 2.1 of *Cladosporium herbarum.* The present invention also relates to assays and kits for detecting allergens.

### BACKGROUND OF THE INVENTION

Allergies to airborne antigens are estimated to afflict 10 - 20% of Europeans and North Americans (Gergen et al, J. Allergy Clin. Immunol., 1987, 80: 669-679). Aeroallergens may consist of plant pollen- or fungal spore-derived protein, or airborne detritus from their biological sources, such as cat dander or dust mites.

The current accepted therapeutic treatment for allergies is to desensitize the patients by injection with extracts prepared from the specific allergen to which the patient is reactive. Ideally, hyposensitization reduces the allergic response through production of IgG antibodies capable of competing for allergen with effector cell-bound IgE, as well as by induction of suppressor T cell populations (Bousquet et al., In: Progress in Allergy and Clinical Immunology, (eds. Pichler et al.) Hogrefe and Huber, Toronto. 1988. pp. 377-382).

Initially, allergen extracts used for desensitization were relatively crude mixtures of protein and carbohydrates extracted from the source organism or material by physicochemical methods. Purification techniques have also been applied to some allergens, yielding partially purified products with improved efficacy (Solvaggio et al., J. Allergy Clin. Immunol. 1993. 92: 217- 222; Mailing et al., Allergy. 1986. 41:507-519). However, commercially prepared allergen extracts are often of inconsistent quality. Impurities or irritants in the extracts are potentially capable of inducing specific IgE production, resulting in risk of anaphylaxis.

The *Alternaria* genus represents one example of the many airborne fungal allergens that can be found indoors. Several species of *Alternaria,* such as *Alternaria alternata,* are known to be plant pathogens and cause hay fever or hypersensitivity reactions that can sometimes lead to asthma. Species of *Alternaria* have also, in rare cases, caused opportunistic infections in immunocompromised patients.

*Cladosporium* is another genus of fungi including some of the most common indoor and outdoor molds. *Cladosporium herbarum* (*C. herbarum*) is one of the major sources of inhalant fungal allergens. One allergenic component present in the *C*. *herbarum* organism is allergen Ch 2.1. This allergen has been cloned, recombinantly produced and characterized, and shown to be a 111 amino acid polypeptide as described in U.S. Patent No. 5,556,953, which is incorporated herein by reference.

A variety of methods have been developed for detection of *Alternaria, Cladosporium* and other aeroallergens, often involving Enzyme-Linked Immunosorbent Assay (ELISA) techniques using antibodies which are specific for allergens of interest. Typically the antibodies are prepared using crude extracts of antigen material, or high abundance native proteins which are purified from the crude material.

### SUMMARY OF THE INVENTION

The object of the invention is to provide an alternative to existing methods and reagents for detecting various fungal allergens, including *A. alternata* and *C*. *herbarum,* as well as methods of preparing polyclonal antibodies that can be used in such methods.

Accordingly, the present invention relates to an isolated polyclonal antibody which specifically binds to recombinantly produced Alt a1 polypeptide, the Alt a1 polypeptide being derived from a cloned source of genetic material.

The invention also relates to an isolated polyclonal antibody which specifically binds to recombinantly produced allergen Ch 2.1 of *C. herbarum,* the Ch 2.1 polypeptide being derived from a cloned source of genetic material.

The present invention also relates to an isolated polyclonal antibody which specifically binds to recombinantly produced target polypeptide, the target polypeptide being derived from a cloned source of genetic material. The target polypeptide may, in certain embodiments, be a fungal allergen, including antigenic polypeptides derived from *A. alternata* and *C*. *herbarum,* although targets may also be derived from other allergen sources.

In addition, the invention also provides a kit comprising a polyclonal antibody as described above, or which is produced according to a method as described below, together with instructions for carrying out an assay for quantifying environmental mould allergens. As an example, the kit may be for detecting and/or quantitating Alt a1, the maj or allergen of *A. alternata.,* or allergen Ch 2.1 of *C. herbarum,* in a sample such as, but not limited to house-hold dust. The kit may thus further comprise a collection device for collecting house-hold dust.

The antibody described above or produced according to a method as described below can be conjugated to a detection reagent, such as a chemoluminescent or colorimetric marker, and provided as an antibody conjugate.

The invention also relates to an immunoassay utilizing the antibody described above or produced according to a method as described below for detecting or quantitating a target polypeptide or allergen. In one non-limiting example for detecting or quantitating *A. alternata* or *C. herbarum,* the immunoassay may comprise the following steps: obtaining a sample suspected of containing *A. alternata* or *C*. *herbarum;* contacting the sample with a polyclonal antibody as described herein, wherein the antibody forms an immune complex with the Alt a1 polypeptide of *A. alternata* or allergen Ch 2.1 of *C*. *herbarum,* if present; contacting the sample with a detection reagent which specifically binds to and forms a complex with said immune complex, and detecting the presence of and/or quantitating the amount of said *A. alternata* or *C*. *herbarum* in said sample based on the amount of said detection reagent bound to said immune complex. In certain non-limiting embodiments, the assay may be an enzyme linked immunosorbant assay (ELISA).

Also provided herein is a process for preparing a polyclonal antibody which specifically binds to a cDNA derived fungal allergen. The process comprising the following steps:
injecting at least one rabbit with about 0.5 - 1 mg of cloned, recombinantly produced fungal allergen protein in saline and complete Freund's Adjuvant (CFA);
after about 14 days from the initial injection, injecting said at least one rabbit with about 0.5 - 1 mg of said cloned, recombinantly produced fungal allergen protein in saline and incomplete Freund's Adjuvant (IFA);
after about 28 days from the initial injection, injecting said at least one rabbit again with about 0.5 - 1 mg of said cloned, recombinantly produced fungal allergen protein in saline and IFA;
after about 42 days from the initial injection, injecting said at least one rabbit again with about 0.5 - 1 mg of said cloned, recombinantly produced fungal allergen protein in saline and IFA;
after about 56 days from the initial injection, injecting said at least one rabbit again with about 0.5 - 1 mg of said cloned, recombinantly produced fungal allergen protein in saline and IFA;
after about 77 days from the initial injection, collecting said polyclonal antibody from said at least one rabbit; and
optionally purifying said polyclonal antibody.

### BRIEF DESCRIPTION OF THE DRAWINGS

These and other features of the invention will become more apparent from the following description in which reference is made to the appended drawings, wherein:
FIGURE 1 shows immunoblots of cloned and native Alt a1 with atopic IgE to *A. alternata.* (A) shows immunoblots of cloned and native Alt a1 run on polyacrylamide gels under non-reducing conditions. (B) shows immunoblots with cloned and native Alt a1 run on polyacrylamide gels under reducing conditions.

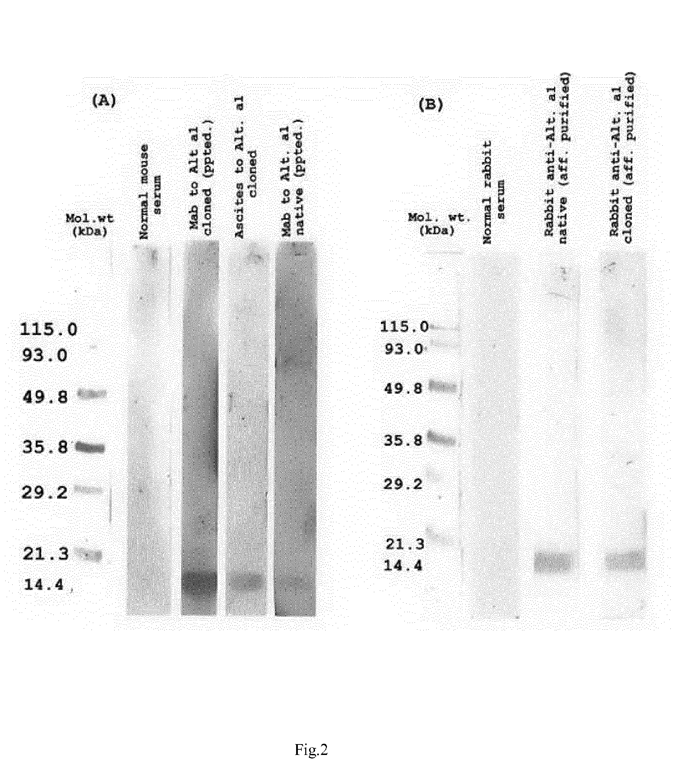

FIGURE 2 shows immunoblots of cloned and native Alt a1 with mouse monoclonal (A) and rabbit polyclonal (B) antibodies.

### DETAILED DESCRIPTION

Described herein are processes for preparing a polyclonal antibody which specifically binds to a cDNA derived fungal allergen, as well as assays and kits for detecting allergens.

In one preferred yet non-limiting embodiment, the invention relates to a major allergen of *Alternaria alternata.* The major allergen of this mold, Alt a1 is a disulfide-linked dimer that migrates in SDS-PAGE under reduced conditions at apparent weight 14,500 and 16,000. Polynucleotide and polypeptide sequences of Alt a1 have been described in the Canadian patent application CA 2,217,173 as SEQ ID NO:3. IgE antibodies to this protein are present in the sera of 90% of *A*. *alternata* sensitive individuals. In a further non-limiting embodiment, the invention also relates to allergen Ch2.1 of *C. herbarum.*

By using recombinant DNA techniques to isolate allergen cDNA clones, allergens may be produced in organisms engineered for large scale protein expression, and conveniently purified with greater fidelity than in the original source. Purified recombinant allergens are of utility in the imunodiagnosis of allergies, and as coating antigens for solid phase assays of atopic sera from allergic individuals, i.e. enzyme-linked immunosorbent (ELISA) or radioallergosorbent (RAST) assays.

Knowledge of the DNA sequences of such allergens may be applied to synthesize short peptide fragments, thus providing a means of localizing IgE-binding or T cell stimulatory epitopes to specific regions of the protein. Desensitization of allergic patients using short peptides comprising such T or B cell epitopes may also be of use as immunotherapeutic reagents administered as part of a desensitization protocol.

Methods used to detect and quantify environmental allergens, such as molds, typically involve ELISA techniques and require large quantities of monoclonal antibodies (Mabs). The production of Mabs is time consuming, and requires sophisticated laboratory facilities. Production of polyclonal antibodies (Pabs), on the other hand, can be accomplished in rabbits with minimal need for equipment and resources. In order to determine whether Pabs can replace Mabs in situations where easy access to Mabs is not possible, Mabs and Pabs to cloned Alt a1, the major allergen of *Alternaria alternata* were produced and their sensitivity and specificity tested.

Progress in the characterization and purification of fungal aeroallergens is complicated by considerable variability with respect to allergen protein profile between strains, as well as difficulties in the recovery of specific spore populations (Vijay et al., Int. Arch. Allergy Appl. Immunol., 1984, 74:256-261; Steringeret al., Int. Arch. Allergy Appl. Immunol., 1987, 84:190-197; Solvaggio et al., J. Allergy Clin. Immunol., 1993, 92:217-222). Because of this variability between isolates of a given species, fewer maj or allergens have been characterized in fungi.

Examination of the sera from individuals allergic to aeroallergens has determined that *Alternaria alternata* represents an important source of fungal aeroallergen (Aas et al., Allergy, 1980, 35:443-451; Yee and Bahna, J. Allergy Clin. Immunol., 1986, 77:200). Initial attempts to characterize the major allergenic components of *A. alternata* extracts yielded protein fractions whose components bound IgE in a majority of *A*. *alternata-*allergic patients (Yunginger et al., J. Allergy Clin. Immunol., 1980, 66:138-147), but were of heterogeneous composition, consisting of several IgE-binding proteins (Vijay et al., Epitopes ofAtopic Allergens. Proceedings of Workshop, XlVth Cong. Eur. Acad. Allergol. Clin. Immunol, Berlin, Sept. 1989; Sehon et al., eds. Brussels, UCB Institute of Allergy, 1990, pp 14-17). Immunoblot analyses of *A. alternata* extracts probed with pooled atopic sera have enabled more precise characterization of individual allergens within fractionated extracts of these. Greater than 90% of atopic sera containing *A. alternata* specific IgE reacted positively against a major allergen purified by several groups and originally designated Alt a-29 (Curran et al., Int. Arch. Allergy Immunol., 1993, 102:267-275), Alt I (Matthiessen et al, Allergologie, 1989, 12:21; Matthiesen et al., J. Allergy Clin. Immunol., 1992, 89:241) or Alt a Bd 29K (Deards and Monague, Mol. Immunol., 1991, 28:409-415). Further purification and immunoblot analysis demonstrated that this allergen, as isolated by the present inventers, is a disulfide-linked dimer of subunits with apparent relative molecular masses of 14,500 and 16,000 (Curran et al., Int. Arch. Allergy Immunol., 1993, 102:267-275) and nearly identical N-terminal sequences that are also 85% homologous to the corresponding N-terminal residues of the isolate of Matthiesen et al. (J. Allergy Clin. Immunol., 1992, 89:241; Vijay et al., J. Allergy Clin. Immunol., 1993, 91:826-828). Based on the striking N-terminal homology between these two protein isolates, these allergens are now considered to represent the same protein, and are collectively termed Alt a1 according to the recently revised system of allergen nomenclature (IUIS/WHO Allergen Nomenclature Subcommittee, Bull World Health Organ., 1994, 72:797-806).

Accordingly, the present invention provides, in a non-limiting embodiment, an isolated polyclonal antibody which specifically binds to recombinantly produced Alt a1 polypeptide, the Alt a1 polypeptide being derived from a cloned source of genetic material. Without wishing to be limiting, it may in certain embodiments be preferred for the antibody to be obtained from rabbit serum. However, production in any mammal is contemplated (for example in goats, mice, rats, etc.).

In the non-limiting embodiment described above, it is preferred that the antibody be specific for *A. alternata,* and therefore it does not specifically bind to Aspergillus, Cladosporium Stachybotrys, and Penicillium allergens by ELISA. Thus, the polyclonal antibody is specific for and is capable of quantifying Alt a1 and/or detecting *A. alternata* in an unknown extract or sample.

In the further embodiment described herein, the antibody may be specific for *C. herbarum, ,* and therefore will not specifically bind to Aspergillus, Alternaria, Stachybotrys, and Penicillium allergens by ELISA. The polyclonal antibody is specific for and is capable of quantifying allergen Ch 2.1 and/or detecting *C. herbarum* in an unknown extract or sample.

Also provided herein, as a further embodiment of the invention, is a kit comprising a polyclonal antibody as described above or produced according to a method as discussed below, together with instructions for carrying out an assay for quantifying environmental mould allergens. Without wishing to be limiting, it may in certain embodiments be preferred for the assay to be an enzyme linked immunosorbant assay (ELISA). In addition, the kit may be for detecting and/or quantitating Alt a1, the major allergen *of A. alternata.,* or allergen Ch 2.1 of *C. herbarum,* e.g. in a sample such as, but not limited to house-hold dust. Thus, in further non-limiting embodiments, the kit may further comprise a collection device for collecting said house-hold dust.

In further embodiments of the invention described herein, the antibody described above or produced according to a method as discussed below can be conjugated to a detection reagent, such as a chemoluminescent or colorimetric marker, and provided as an antibody conjugate.

Also provided herein is an immunoassay. In certain non-limiting embodiments, the immunoassay may be for detection or quantitation of the Alt a1 polypeptide or allergen Ch 2.1, and/or *A. alternata* or *C. herbarum,* and comprise the following steps: obtaining a sample suspected of containing *A. alternata* or *C. herbarum*,; contacting the sample with a polyclonal antibody as described herein, wherein the antibody forms an immune complex with the Alt a1 polypeptide of *A. alternata* or allergen Ch 2.1 of *C. herbarum,,* if present; contacting the sample with a detection reagent which specifically binds to and forms a complex with said immune complex, and detecting the presence of and/or quantitating the amount of said *A. alternata* or *C*. *herbarum* in said sample based on the amount of said detection reagent bound to said immune complex. In certain non-limiting embodiments, the assay may be an enzyme linked immunosorbant assay (ELISA).

Also provided herein is a process for preparing a polyclonal antibody which specifically binds to a cDNA derived fungal allergen. The process comprising the following steps:
injecting at least one rabbit with about 0.5 - 1 mg of cloned, recombinantly produced fungal allergen protein in saline and complete Freund's Adjuvant (CFA);
after about 14 days from the initial injection, injecting said at least one rabbit with about 0.5 - 1 mg of said cloned, recombinantly produced fungal allergen protein in saline and incomplete Freund's Adjuvant (IFA);
after about 28 days from the initial injection, injecting said at least one rabbit again with about 0.5 - 1 mg of said cloned, recombinantly produced fungal allergen protein in saline and IFA;
after about 42 days from the initial injection, injecting said at least one rabbit again with about 0.5 - 1 mg of said cloned, recombinantly produced fungal allergen protein in saline and IFA;
after about 56 days from the initial injection, injecting said at least one rabbit again with about 0.5 - 1 mg of said cloned, recombinantly produced fungal allergen protein in saline and IFA;
after about 77 days from the initial injection, collecting said polyclonal antibody from said at least one rabbit; and
optionally purifying said polyclonal antibody.

In certain non-limiting embodiments, the cloned, recombinantly produced fungal allergen protein is recombinantly produced Alt a1 protein, and the polyclonal antibody will specifically bind to cloned Alt a1 protein from *A*. *alternata.*

In a further non-limiting embodiment, the cloned, recombinantly produced fungal allergen protein is recombinantly produced allergen Ch 2.1, and the polyclonal antibody will specifically bind to cloned Ch 2.1 protein from *C*. *herbarum.* Further non-limiting embodiments will become apparent from the following Examples.

### EXAMPLES:

### Example 1: Culture of Alternaria alternata 34-016

A 34-016 isolate of *A*. *alternata* was grown on synthetic revised tobacco medium (RTM) as reported earlier (Vijay HM, et al., Grana, 1989, 28:53-61). Briefly, the cultures were incubated at 22 °C for 117 days. The mycelia and spores were separated and the broths were decanted from the cultures and centrifuged at 30,000 x g for 20 min. at 4 °C. The supernatant was concentrated by ultrafiltration using Amicon YM5 membrane. The retentate (5kDa) was washed exhaustively with water, filtered (0.45 µ) and lyophilized.

### Example 2: Preparation Of Native Alt a1

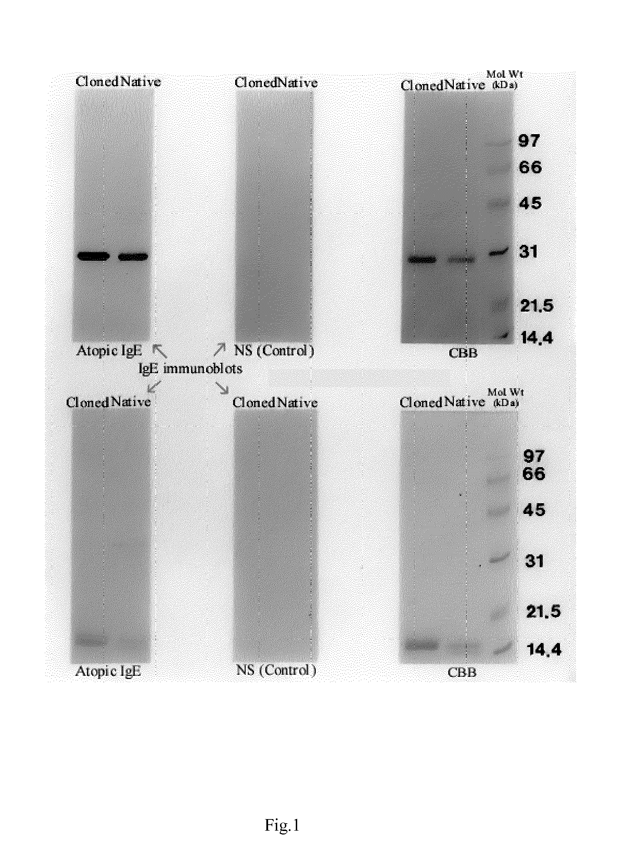

Native Alt a1 was purified from lyophilized extract of *A. alternata* using gel filtration, i.e. Sephadex column chromatography techniques (Curran IHA, et al., Int. Arch. Allergy Immunol, 1993, 102:267-275). Purified protein was characterized by SDS-PAGE and immunoblotting with atopic serum to *A. alternata* (see Fig. 1).

### Example 3: Preparation Of Recombinant Alt a1

*A. alternata* was cultured in RTM as described in Example 1. From a 7 day old culture, the RNA was isolated using guanidine isothiocynate (DeVouge N.M. et al., Int. Archs. Allergy Immunol., 1996, 385-395). Preparation of a cDNA library in λgt11 was carried out by a commercial firm (Clonetech CA).

### Immuno-screening of the cDNA Library

Polyclonal rabbit anti-serum was prepared against native Alt a1 that was partially purified with Whatman DE-52 ion exchange chromatography followed by Sephadex G-100 gel filtration. This antiserum was used in immune-screening and immunoblots. In order to detect allergenicity of the clones, the library was screened using pooled atopic sera to *A. alternata.* The cDNA encoding Alt a-29 (Alt a1) was cloned and expressed in *Pichia pastoris* and purified by FPLC.

### Example 4: Production of Polyclonal Antibodies to Cloned and Native Alt a1

Antibodies were produced to cloned/native Alt a1 by immunizing male New Zealand white rabbits by subcutaneous (s.c.) injection with emulsion prepared from 0.4 ml of saline containing 725 µg of cloned/native Alt a1 and 0.4 ml complete Freund's adjuvant (CFA). Two weeks later, the animals received the same dose of antigen in 0.4 ml incomplete Freund's adjuvant (IFA). Seven weeks later, trial bleeding and booster shots were made at weekly intervals. The protocol is outlined in Table 1 below.

**Table 1: Immunization Protocol for Raising Polyclonal Antibodies to Alt a1. Duration 77 days.**

| **Day** | **Injection** | **Bleeding** |
|---|---|---|
| 0 | | 10 cc prebleed |
| 0 | Primary injection of 725 µg cloned/native Alt a1 in 0.4 ml saline + 0.4 ml CFA (s.c.- 8 sites on the dorsal side | - |
| 14 | Boost (1) Injection (2) Exactly the same as in (1) except this time 725 µg cloned/native antigen in 0.4 ml saline + 0.4 ml IFA (8 sites on the dorsal side) | - |
| 21 | | 10 cc prebleed |
| 28 | Boost (2) Injection (3) same as in boost (1) | - |
| 35 | - | Trial bleed 10 cc |
| 42 | Boost (3) Injection (4) same as in (1) | - |
| 49 | - | Trial bleed 10 cc |
| 56 | Boost (4) Injection (5) same as in (1) | - |
| 63 | - | Trial bleed 10 cc check by ELISA or ID |
| 70 | - | |
| 77 | - | Terminal bleed of the animals according to animal research division guidelines |

### Example 5: Production of IgG Antibodies to Cloned Alt a1 to Achieve Monoclonal Antibodies

Balb/c mice were pre-bled prior to immunization with the cloned Alt a1. The animals were then given subcutaneous injections on the dorsal side with 30 µg of lyophilized protein antigen in 0.05 ml saline emulsified with 0.05 ml Complete Freund's adjuvant; 0.05 ml were injected at two sites. 28 days later a booster injection was given in a similar way with the same dose of antigen emulsified with Incomplete Freund's adjuvant followed by a second booster injection at day 56 (exactly the same as the first booster injection). 7 days later, i.e., day 63, the animals were trial bled. Similarly, the animals were given the third booster injection at day 84. 7 days later, i.e. day 91, the animals were trial bled. At this stage, the antisera were tested by ELISA and RAST. If the titer was adequate, the animals were given the fourth booster injection at day 112, intravenously with the same dose of the antigen in 0.1 ml of saline. 3 days later, i.e. day 115, the 4 animals were bled and the titer of the antisera examined by ELISA. 3 days later, i.e. day 118, after a trial bleed, the spleens were removed from two mice giving the highest titer of the antibody. The spleen cells were fused with the mouse myeloma cell lines to obtain hybridomas. The hybridomas were grown *in vitro* in order to produce the desired monoclonal antibodies. Total duration of immunization was 118 days. The protocol is outlined in Table 2 below.

**Table 2: Immunization Protocol for Producing IgG Antibodies to Alt a1. Duration 118 days.**

| Day | Injection | Trial Bleed |
|---|---|---|
| 0 | Bleed the animals prior to immunization (Control sera) (8 mice per antigen) the mice are weighed at least once/week | Pre bleed 0.5 ml per mouse |
| 0 | Administer the primary immunization by subcutaneous (SC) injection on the dorsum of each mouse. Mix 30 µg of the lyophilized protein antigen in 0.05 ml of sterile saline emulsified with 0.05 ml of complete Freund's Adjuvant (CFA). Ensure that the solution is well mixed immediately prior to injecting 0.05 ml of the mixture SC at each of 2 sites on the dorsum of the mouse. | - |
| 28 | Administer the 1^{st} booster injection to each mouse. Mix 30µg of antigen in 0.05 ml of sterile saline emulsified with 0.05 ml of Incomplete Freund's Adjuvant (IFA). Ensure that the solution is well mixed immediately prior to injecting 0.05 ml of the mixture SC at each of 2 sites on the dorsum of the mouse. | - |
| 56 | Administer of the 2^{nd} booster injection to each mouse and follow the procedure from day 28. | - |
| 63 | - | Collect 50 µl of whole blood from each anesthetized mouse, via the retro-orbital sinus of the eye, for evaluation of the titer level. Recover each mouse after blood collection is complete. |
| 84 | Administer the third booster injection to each mouse by following the procedure from day 28 | - |
| 91 | | Collect 50µl of whole blood from each anesthetized mouse via the retro-orbital sinus of the eye, for evaluation of the titer levels. Recover each mouse after blood collection is complete. |
| 112 | If the titer levels are adequate, administer the fourth booster injection intravenously via the tail vein in the conscious mouse. Mix 30 µg of the antigen in 0.1 ml of sterile saline. Ensure that the solution is well mixed immediately prior to injecting the mixture IV into the tail vein of the mouse. | - |
| 115 | | Collect 50 µl of whole blood from each anesthetized mouse via the retro-orbital sinus of the eye, for evaluation of titer levels. Recover each mouse after blood collection is complete. |
| 118 | | Exsanguinate each anesthetized mouse via cardiac puncture. Remove the spleens from only those mice demonstrating the highest titer levels of antibody. The spleen cells are to be fused with the mouse myeloma cell lines to obtain hybridomas *in vitro* to produce monoclonal antibodies |

### Example 6: Isotyping of mAbs to Cloned and Native Alt a1

Isotyping was carried out using a Sigma™ isotyping kit. In brief, isotyping sticks were immersed in 2 ml of a 1:2 dilution of the hybridoma supernatant and incubated for 30 min. and washed once in 1 x PB S-T. Two ml of a 1:50 dilution of biotinylated anti-mouse antibody was added and incubated for 5 min., followed by washing once with 1 x PBS-T/BSA for 5 min. Two ml of a 1:50 streptavidin-peroxidase was added and incubated for 5 min. This was followed by a wash in 1 x PBS-T, followed by another wash in 1 x PBS-T, followed by a wash with PBS. The results were then observed.

### Example 7: Production of Ascites

Balb/c mice were primed with Pristane 7-10 days prior to injection with the hybridoma cells, and 1 x 10⁶ cloned cells in 0.5 ml serum-free Dulbecco's modified Minimum essential Medium (D-MEM) were injected per mouse. Seven days later, the mice were examined for tumors, and ascites fluid was collected.

### Example 8: Specificity of the Polyclonal and Monoclonal Antibodies to Cloned and Native Alt a1

Immulon 2B microtiter plates were coated with cloned/native Alt a1 at 100 ng/well in PBS pH 7.4 and kept at 4 °C overnight. The plate was washed with PBS-T twice and blocked with 0.2 ml 1 % BSA/PBS per well and incubated at room temperature. The plate was washed with PBS-T 4 times and 0.1 ml of the test antibodies were dispensed per well and incubated for 1 hr at room temperature. The monoclonal antibodies (Mabs) were diluted 1:10, 1:50, and 1:100 and the polyclonal antibodies (Pabs) 1:1000. The plate was then washed with PBS-T. Peroxidase labelled antibody was added (1:10,000) at 0.1 ml/well and incubated for 1 hr at room temperature. The plate was washed 5 times and the reaction was developed by adding TMB peroxidase substrate system (KLP50-7500) and the OD was read at (450nm).

### Results:

The antibodies to both native and cloned Alt a1 were observed to belong to the IgG2b sub-class. The rabbit polyclonal and mouse monoclonal antibodies did not show any cross-reactivity when examined against *Aspergillus, Cladosporium, Stachybotrys,* and *Penicillium* allergens by ELISA. Also, polyclonal antibodies against cloned or native Alt a1 were found to be specific in quantifying the major allergen in an unknown *A. alternata* extract and correlated well with the monoclonal antibodies. The findings suggest that polyclonal antibodies have the desired specificity and can be used to develop ELISA kits for quantifying environmental mould allergens.

Accordingly, polyclonal antibodies against cloned and native Alt a1 can be used to develop ELISA kits for detection of *A*. *alternata.* This is especially beneficial since purified polyclonal antibodies to cloned and native Alt a1 can easily be obtained in larger quantities as compared to monoclonal antibodies, and without the need for a cell culture facility.

One or more currently preferred embodiments have been described by way of example. It will be apparent to persons skilled in the art that a number of variations and modifications can be made without departing from the scope of the invention as defined in the claims.

## Claims

1. An isolated polyclonal antibody which specifically binds to a recombinantly produced target polypeptide, the target polypeptide being derived from a cloned source of genetic material.

2. The isolated polyclonal antibody of claim 1, wherein the recombinantly produced target polypeptide is Alt a1 from *A. alternata,* or an antigenic fragment thereof, and the polyclonal antibody specifically binds to said Alt a1 polypeptide, said Alt a1 polypeptide or antigenic fragment thereof being derived from a cloned source of *A. alternata* genetic material, or wherein the recombinantly produced target polypeptide is allergen Ch 2.1 from *C. herbarum,* or an antigenic fragment thereof, and the polyclonal antibody specifically binds to said allergen Ch 2.1, said allergen Ch 2.1 or antigenic fragment thereof being derived from a cloned source of *C. herbarum* genetic material.

3. The polyclonal antibody of claim 1, wherein said antibody is obtained from rabbit serum.

4. The polyclonal antibody of claim 2 or 3, wherein said antibody does not bind to Aspergillus, Stachybotrys, and Penicillium allergens by ELISA.

5. The polyclonal antibody of claim 1, wherein said antibody specifically binds to and is capable of quantifying Alt a1 and/or detecting *A. alternata* in an unknown extract or sample.

6. The polyclonal antibody of claim 1, wherein said antibody specifically binds to and is capable of quantifying allergen Ch 2.1 and/or detecting *C. herbarum* in an unknown extract or sample.

7. A kit comprising a polyclonal antibody as defined in any one of claims 1 to 6 and instructions for carrying out an assay for quantifying environmental mould allergens.

8. The kit of claim 7, wherein said assay is an enzyme linked immunosorbant assay (ELISA).

9. The kit of claim 7 or 8, wherein said kit is for detecting and/or quantitating Alt a1, the major allergen of *A. alternata,* or wherein said kit is for detecting and/or quantitating allergen Ch 2.1, the major allergen of *C. herbarum.*

10. The kit of any one of claims 7 to 9, wherein said kit is for detecting said allergen in house-hold dust, and optionally further comprising a collection device for collecting said house-hold dust.

11. An antibody conjugate comprising the antibody of any one of claims 1 to 6, covalently linked to a detection reagent.

12. An immunoassay comprising the steps of
obtaining a sample suspected of containing *A. alternata* and/or *C. herbarum;*
contacting said sample with at least one antibody of claim 1, wherein said antibody forms an immune complex with the Alt a1 polypeptide of *A*. *alternata* and/or allergen Ch 2.1, of *C. herbarum,* if present,
contacting said sample with a detection reagent which specifically binds to and forms a complex with said immune complex, and
detecting the presence of and/or quantitating the amount of said *A. alternata* and/or *C. herbarum* in said sample based on the amount of said detection reagent bound to said immune complex.

13. The immunoassay of claim 12, wherein said immunoassay is an enzyme linked immunosorbant assay (ELISA).

14. A process for preparing a polyclonal antibody which specifically binds to a cDNA derived fungal allergen, said process comprising the steps of
injecting at least one rabbit with about 0.5 - 1 mg of cloned, recombinantly produced fungal allergen protein in saline and complete Freund's Adjuvant (CFA)
after about 14 days from the initial injection, injecting said at least one rabbit with about 0.5 - 1 mg of said cloned, recombinantly produced fungal allergen protein in saline and incomplete Freund's Adjuvant (IFA);
after about 28 days from the initial injection, injecting said at least one rabbit again with about 0.5 - 1 mg of said cloned, recombinantly produced fungal allergen protein in saline and IFA;
after about 42 days from the initial injection, injecting said at least one rabbit again with about 0.5 - 1 mg of said cloned, recombinantly produced fungal allergen protein in saline and IFA;
after about 56 days from the initial injection, injecting said at least one rabbit again with about 0.5 - 1 mg of said cloned, recombinantly produced fungal allergen protein in saline and IFA;
after about 77 days from the initial injection, collecting said polyclonal antibody from said at least one rabbit; and
optionally purifying said polyclonal antibody.

15. The process of claim 14, wherein said polyclonal antibody specifically binds to cloned Alt a1 protein from *A. alternata,* and said cloned, recombinantly produced fungal allergen protein is recombinantly produced Alt a1 protein, or wherein the polyclonal antibody specifically binds to cloned allergen Ch 2.1 of *C. herbarum,* and said cloned, recombinantly produced fungal allergen protein is recombinantly produced allergen Ch 2.1.
